(19) Europäisches Patentamt
European Patent Office
Office européen des brevets

(11) Numéro de publication: **0 138 632**
**B1**

# (12) FASCICULE DE BREVET EUROPEEN

(45) Date de publication du fascicule du brevet: **02.01.91**

(51) Int. Cl.[5]: **C 08 B 37/10,** C 07 K 9/00, A 61 K 31/725

(21) Numéro de dépôt: **84401396.1**

(22) Date de dépôt: **02.07.84**

## (54) Complexes contenant des oligosaccharides, leur préparation et leurs applications biologiques et biochimiques.

(30) Priorité: **01.07.83 FR 8311004**

(43) Date de publication de la demande: **24.04.85 Bulletin 85/17**

(45) Mention de la délivrance du brevet: **02.01.91 Bulletin 91/01**

(84) Etats contractants désignés: **AT BE CH DE FR GB IT LI LU NL SE**

(56) Documents cités:
**EP-A-0 027 089 EP-A-0 084 999**

**CARBOHYDRATE RESEARCH, vol. 100, nos. 1/2, mars 1982, pages 393-410, Elsevier Scientific Publishing Co., Amsterdam, NL; L. THUNBERG et al.: "Further characterization of the antithrombin-binding sequence in heparin"**

**THE JOURNAL OF BIOLOGICAL CHEMISTRY, vol. 254, no. 8, 25 avril 1979, pages 2902-2913, US; R. JORDAN et al.: "Fractionation of low molecular weight heparin species and their interaction with antithrombin"**

**Le dossier contient des informations techniques présentées postérieurement au dépôt de la demande et ne figurant pas dans le présent fascicule.**

(73) Titulaire: **CHOAY S.A.
48, Avenue Théophile-Gautier
F-75782 Paris Cédex 16 (FR)**

(72) Inventeur: **Petitou, Maurice
27, rue du Javelot
F-75645 Paris Cedex 13 (FR)**
Inventeur: **Lormeau, Jean-Claude
1, rue Joseph Delattre
F-76150 Maromme (FR)**
Inventeur: **Choay, Jean
21, rue Saint-Guillaume
F-75007 Paris (FR)**

(74) Mandataire: **Peaucelle, Chantal et al
S.C. Ernest Gutmann - Yves Plasseraud 67, boulevard Haussmann
F-75008 Paris (FR)**

(56) Documents cités:
**BIOCHEMICAL JOURNAL, vol. 193, 1981, pages 427-433, The Biochemical Society, GB; A. DANIELSSON et al.: "Binding to antithrombin of heparin fractions with different molecular weights"**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

EP 0 138 632 B1

**Description**

L'invention est relative à de nouveaux complexes contenant des oligosaccharides, ces derniers possédant d'une activité spécifique anti-Xa élevée.

Elle concerne également leur obtention et leurs applications biologiques et biochimiques.

Les oligosaccharides des dérivés correspondent ou comprennent des fragments de mucopolysaccharides acides possédant la structure de ceux rencontrés dans les chaînes de dérivés biologiquement actifs du type de l'héparine ou de l'héparane-sulfate.

Dans ces produits naturels, les mucopolysaccharides en question sont formés de motifs alternés sucre aminé-acide uronique ou inversement.

Le sucre aminé, désigné ci-après par *a*, présente plus spécialement une structure D-glucosamine et l'acide uronique, qu'on appellera *U* présente une structure *b* de type acide D-glucuronique ou *c* de type acide L-iduronique.

Les structures de base pour *a, b* et *c* sont les suivantes:

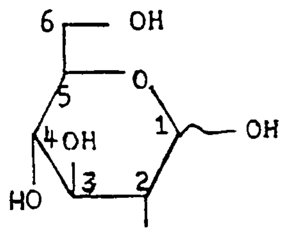

dérivé d'aminé

(a) D-glucosamine

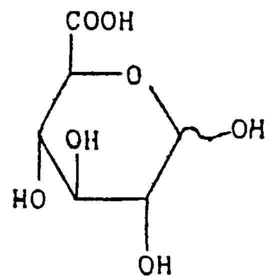

(b) acide D-glucuronique

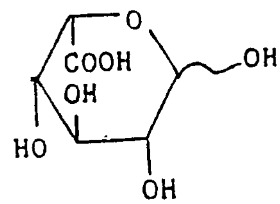

(c) acide L-iduronique.

Ces différents motifs sont liés entre eux de manière stéréospécifique dans les produits naturels. Ainsi on trouve, par exemple dans l'héparine des liaisons de type

$\overset{\alpha}{1\text{—}4}$ (entre les motifs *c* et *a*, *a* et *b*, et *a* et *c*) et de type $\overset{\beta}{1\text{—}4}$

(entre les motifs *b* et *a*).

Grâce au procédé de synthèse par voie organique des oligosaccharides en question mis au point par la Demanderesse et qui fait l'objet notamment de la demande FR 82 18003 du 27 octobre 1982, il est à présent possible de disposer aisément de ces produits, sous une forme extrêmement pure et ce, selon l'enchaînement, la configuration et les substitutions souhaitées.

Cette disponibilité a permis de progresser d'une manière générale dans la connaissance de la relation de l'activité de fragments de molécules biologiquement actives à leur structure.

On sait que l'héparine et différents fragments de celle-ci inhibent un certain nombre de protéases procoagulantes essentiellement en potentialisant l'activité de l'ATIII sur laquelle ils se fixent.

Sur la base de différents travaux effectués, les inventeurs ont été amenés à considérer que la séquence oligosaccharidique minimale pour obtenir une fixation à l'ATIII était constituée par une séquence de 5 motifs sucre correspondant à un enchaînement, en ce qui concerne la structure de base, de type a-b-a-c-a.

L'etude approfondie d'un pentasaccharide de ce type a permis d'établier qu'il augmentait in vitro et in vivo l'activité inhibitrice de l'ATIII vis-à-vis du facteur Xa de la coagulation sanguine, l'activité anticoagulante globale étant nulle (les mesures d'activité anti-Xa ont été effectuées selon la méthode de Yin et Wessler décrite par ces auteurs dans J. Lab. Clin. Med. 1976, 81, 298—300, et une méthode modifiée de Teien et al dans Thrombosis Research n° 10, 1977, 388—410).

L'activité anticoagulante globale a été mesurée selon le test USP décrit dans US Pharmacopea XX p. 915.

Les inventeurs ont alors constaté qu'en mettant à profit l'affinité pour l'ATIII d'oligosaccharides à activité spécifique anti-Xa élevée, il était possible notamment d'augmenter encore cette activité.

L'invention a donc pour but de fournir de nouveaux complexes contenant des oligosaccharides, ces derniers possédant une activité spécifique anti-Xa élevée.

Elle a également pour but de fournir un procédé d'obtention aisée de ces complexes.

Elle vise également leurs applications biologiques et biochimiques, en particulier la mise à profit de leur forte activité anti-Xa spécifique pour l'élaboration de médicaments à activité antithrombotique.

Les complexes contenant des oligosaccharides à activité anti-Xa élevée sont caractérisés en ce qu'il s'agit de complexes à base d'ATIII et d'oligosaccharides de formule:

$$X\,(aU)_n\,Y$$

dans laquelle:

X est un groupe —OH ou un acide uronique soit de type acide D-glucuronique, soit de type acide L-iduronique,

Y est un groupe —OH ou un motif D-glucosamine,

a est un motif D-glucosamine,

U est un motif acide uronique soit b de type acid D-glucuronique, soit c de type acide L-iduronique,

n est un entier de 2 à 6, le nombre total de motifs saccharidiques étant de 5 à 12.

Ces complexes résultent de la simple affinité d'un oligosaccharide donné pour l'ATIII. Ils sont réversibles, pouvant se dissocier et se ré-associer selon les conditions auxquelles ils sont soumis, ce qui les distinguent donc de dérivés tels que conjugués faisant intervenir une liaison chimique covalente irréversible.

Il s'avère que dans les complexes de l'invention l'activité spécifique anti-Xa est considérablement augmentée, ce qui permet de disposer de produits possédant une forte activité antithrombotique.

Selon une variante, l'alternance régulière des motifs *a* et *U* est modifiée par la présence à la suite d'un ou plusieurs motifs *a* ou *U* et/ou d'un ou plusieurs motifs de sucres neutres et/ou de désoxy-sucres et/ou de sucres de configuration différente.

Une famille préférée de complexes de l'invention comprend des oligosaccharides comportant ou correspondant, en totalité ou en partie, à une séquence octasaccharidique de type b-a-c-a-b-a-c-a.

Dans cette séquence, les groupes alcool primaire et alcool secondaire peuvent être libres. En variante, certains d'entre eux ou la totalité peuvent être substitués par des anions minéraux ou organiques.

Les anions phosphate et plus spécialement sulfate sont particulièrement préférés, notamment sous forme de sels avec un cation minéral ou organique, en particulier un cation métallique, notamment un cation alcalin, ou encore un cation dérivé d'une base organique azotée, par exemple triéthylammonium. Des cations préférés sont constitués par du sodium. D'autres cations appropriés comprennent le potassium, le magnésium ou le calcium.

Les groupes amino des motifs *a* peuvent être libres ou substitués avec, dans ce cas, des substitutions identiques ou différentes.

Des groupes de substitution préférés comprennent les groupes acyle, tels qu'acétyle ou benzyle ou des anions tels que ceux évoqués ci-dessus, en particulier, l'anion $SO_3^-$. Ces anions se présentent avantageusement sous forme de sel avec un cation tel que défini ci-dessus.

Les groupes carboxyle des motifs *b* ou *c* sont libres ou se présentent de préférence sous forme de sels avec un cation organique ou minéral comme défini plus haut.

Des produits préférés renferment des motifs *c* comportant un group sulfate en position 2.

D'autres produits préférés renferment, éventuellement en plus des dispositions qui précèdent, un motif *a* avec un groupe 3 et/ou 6-0-sulfate.

Des produits préférés ont un groupe *a* N-substitué, notamment N-sulfate avec un groupe 3 et/ou 6-0-sulfate.

Compte tenu de leur présence, en particulier, dans l'héparine, des oligosaccharides préférés comprennent en partie ou en totalité la séquence octasaccharidique de structure ABCDEFGH décrite par les inventeurs, notamment dans le brevet EP 0 027 089 répondant à la formule:

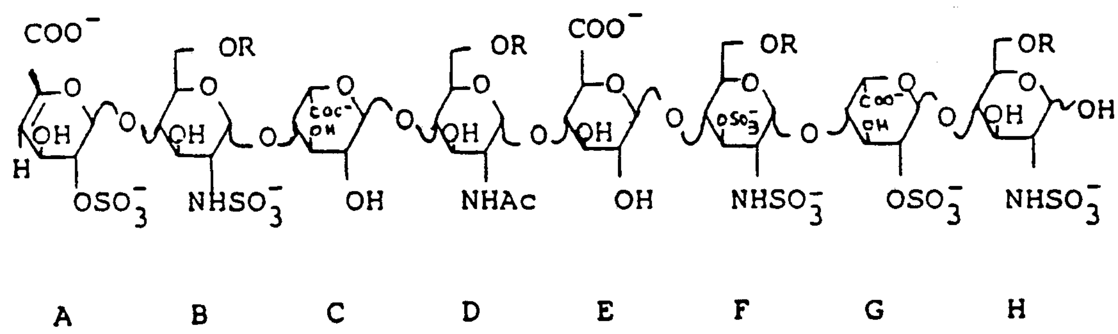

dans laquelle R représente un groupe —OH ou —$SO_3^-$.

Il est entendu qu'il s'agit là d'une structure de base et que des produits équivalents peuvent comporter

des substituants différents de ceux indiqués dans la formule. En particulier, pour le motif D, le substituant en position 2 peut être un groupe —NHR′ avec R′ représentant H, un groupe acyle, en particulier acétyle ou —$SO_3^-$.

Les références à ces motifs donnés ci-après concernent donc avant tout leur structure.

Des hexasaccharides, notamment de structure CDEFGH sont avantageusement utilisés dans les complexes de l'invention.

Des produits, particulièrement préférés, étant donné qu'ils correspondent selon toute vraisemblance à la séquence minimale requise pour la fixation à l'ATIII, et qu'ils sont doués d'une activité anti-Xa spécifique élevée, sont constitués par les pentasaccharides de structure du type a-b-a-c-a sous forme de complexe avec l'ATIII.

Des complexes tout spécialement préférés renferment une séquence de type DEFGH.

Dans ces complexes, la séquence pentasaccharidique peut être substituée comme indiqué sur la formule ci-dessus, ou comporter des substituants différents, en particulier au niveau des groupes —$OSO_3^-$ qui peuvent être remplacés par des groupes tels que des groupes —OH ou au niveau des groupes amino.

En particulier, les groupes amino des motifs D, F et H sont avantageusement identiques. Ils peuvent représenter, par exemple, un groupe —NH—$SO_3^-$.

Dans les complexes ci-dessus, on utilise avantageusement de l'ATIII humaine, compte tenu des applications biologiques envisagées chez l'homme.

Conformément à l'invention, les complexes définis ci-dessus sont obtenus par simple mélange, de quantités équimolaires d'ATIII et d'oligosaccharides dans un milieu tampon.

Ce mélange est laissé à température ambiante. Une durée d'environ 15 mn apparaît généralement suffisante pour l'obtention du complexe. Ce dernier peut être récupéré par gel filtration. L'utilisation de tampon d'élution à forte concentration en sels conduit à la dissociation du complexe.

On mesurera l'intérêt de la mise en oeuvre dans ce procédé d'oligosaccharides obtenus par voie de synthèse, selon le procédé décrit précédemment par la demanderesse, ce qui permet de disposer des produits désirés avec une grande pureté.

L'étude des complexes ATIII-oligosaccharides de l'invention ont permis de montrer diverses de leurs caractéristiques: des vérifications expérimentales, notamment par filtration sur gel (opération dénommée ci-avant gel filtration) et études de fluorescence exploitée selon la méthode de Scatchard, montrent que ces complexes sont équimolaires et possèdent une forte constante d'association de $10^5$ à $10^7$ $mole^{-1}$ environ.

On a également constaté que ces complexes ATIII-oligosaccharides présentent une activité anti-Xa supérieure à celle de l'oligosaccharide seul. En raison de son engagement dans le complexe, l'oligosaccharide est avantageusement protégé de l'action des autres protéines et agit plus rapidement.

Lorsque l'ATIII présente dans le complexe est engagée dans l'inhibition du facteur Xa, l'oligosaccharide se trouve libéré; il peut ainsi de nouveau se lier à l'ATIII présente normalement dans la plasma et promouvoir ainsi de façon répétitive de nouvelles quantités du facteur Xa.

On mesurera l'intérêt de cet apport en ATIII pour des patients souffrant d'une déficience en ATIII ou lorsqu'il s'agit de malade en état d'hypercoagulabilité ou d'hypercoagulation notamment en état de coagulation intra-vasculaire disséminé ou souffrant d'une thrombose aiguë, tous états nécessitant un traitement d'attaque très rapide. Le traitement par les complexes de l'invention permettant de ne pas dépendre du taux d'ATIII du malade.

Cet apport en ATIII et sa meilleure utilisation permettent également d'éviter les déficiences provoquées en ATIII par des traitements de longue durée avec de l'héparine.

De plus l'activité anti-Xa de l'oligosaccharide aura une demi-vie plus longue lorsque ce dernier est engagé dès le départ sous forme d'un complexe puisque l'oligosaccharide échappe de ce fait aux enzymes et à des protéines neutralisantes.

Des complexes préférés renferment, en tant qu'oligosaccharide, une séquence constituant la copie, tout au moins en ce qui concerne la structure, des séquences naturelles présentes dans l'héparine.

Il s'agit, en particulier, de complexes ATIII-hexasaccharides ou encore ATIII-pentasaccharides de structure du type des chaînes CDEFGH et DEFGH définies ci-dessus.

Les complexes ATIII-pentasaccharides définis ci-dessus présentent un intérêt tout particulier étant donné que le pentasaccharide renferme la séquence considérée, d'après les résultats obtenus, comme étant la séquence minimale pour la fixation à l'ATIII.

L'activité anti-Xa de ces complexes est au moins trois fois plus importante environ que celle du pentasaccharide utilisé seul, atteignant des valeurs de l'ordre de 9000 u/mg (unité YW) pour le complexe ATIII-pentasaccharide de type DEFGH.

Des résultats semblables sont également obtenus dans le test amidolytique de Teien modifié en utilisant du tampon tris-maléate (0.02M) pH 7,5 au lieu de tampon tris-HCl pour la dilution du plasma.

Selon un aspect d'un intérêt considérable, cette activité anti-Xa est totalement spécifique vis-à-vis du facteur Xa. En effet, l'activité anti-thrombine, vis-à-vis du facteur IIa, est nulle comme démontré par les résultats obtenus en effectuant l'essai amidolytique de Larsen M-L Abildgaard U, Teien A. M. et Gjesdalk K. (1978), Thromb. Res. 13, 285—288.

Ces complexes qui sont, en outre, dépourvus de toxicité sont donc avantageusement utilisables pour la fabrication de médicaments utilisables notamment pour la prévention et le traitement des thromboses.

L'invention est donc relative également à des préparations pharmaceutiques qui renferment lesdits

complexes ATIII-oligosaccharides à activité spécifique anti-Xa élevée, plus spécialement les complexes ATIII-pentasaccharides dont question ci-dessus.

Elle est plus particulièrement relative à des préparations pharmaceutiques, dépourvues de substances pyrogènes, contenant une quantité efficace de principes actifs en association avec des excipients pharmaceutiques, notamment sous forme lyophylisée.

Elle concerne également les compositions dans lesquelles la véhicule pharmaceutique est approprié pour l'administration par voie orale. Des formes d'administration de l'invention appropriées pour l'administration par voie orale peuvent être avantageusement des gélules gastrorésistantes, des comprimés ou tablettes, des pilules, ou encore présentées sous forme de liposomes.

D'autres compositions pharmaceutiques comprennent ces oligosaccharides en association avec les excipients appropriés pour l'administration par voie rectale. Des formes d'administration correspondantes sont constituées par des suppositoires.

D'autres formes d'administration de l'invention sont constituées par des aérosols ou des pommades.

L'invention concerne également des compositions pharmaceutiques injectables, stériles ou stérilisables pour l'administration tant par voie intraveineuse qu'intramusculaire ou sous-cutanée.

Ces solutions renferment avantageusement 1 000 à 50 000 u(Yin-Wessler)/ml de préférence de 1 500 à 30 000 par exemple de 25 000 u/ml, lorsque ces solutions sont destinées à l'injection par voie intraveineuse ou sous-cutanée. A titre d'exemple de compositions pharmaceutiques, on peut utiliser des solutions du complexe dans du soluté chlorure isotonique à la concentration de 25 000 unités anti-Xa par ml, la dose unitaire envisagée pouvant aller de 5 000 à 50 000 unités, par exemple, étant de 25 000 unités anti-Xa par ml.

Avantageusement, de telles préparations pharmaceutiques sont présentées sous la forme de seringues non récupérables prêtes à l'emploi.

Les compositions pharmaceutiques de l'invention sont particulièrement adaptées pour le contrôle (préventif ou curatif) de certaines étapes de la coagulation du sang chez l'homme ou l'animal, notamment dans le cas où le patient est soumis à des risques d'hypercoagulabilité résultant notamment d'opérations chirurgicales, de processus athéromateux, de développment de tumeurs et de troubles de la coagulation par des activateurs bactériens ou enzymatiques, etc.... en particulier dans le cas de déficiences en ATIII.

Afin d'illustrer l'invention, on indique ci-après, un exemple de posologie utilisable chez l'homme: cette posologie comprend, par exemple, l'administration au patient de 5 000 à 50 000 u(Yin et Wessler) d'oligosaccharide engagé dans le complexe par voie intraveineuse, par exemple de 8 000 à 30 000, en une ou plusieurs injections, en administrations discontinues à intervalles réguliers, ou continues par perfusion, ou encore tous les deux ou trois jours, selon le niveau des risques d'hypercoagulabilité ou la condition thrombotique du patient. Ces doses peuvent être naturellement ajustées pour chaque patient en fonction des résultats et des analyses de sang effectuées auparavant, la nature des affections dont il souffre et, d'une manière générale, son état de santé.

D'autres caractéristiques et avantages de l'invention apparaîtront dans les exemples qui suivent.

Exemple 1

Préparation d'un complexe d'ATIII humaine et de pentasaccharide de formule:

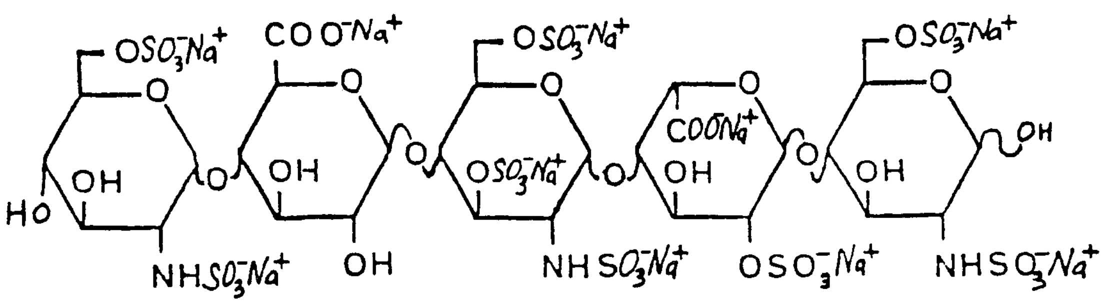

(50)

On mélange 65 mg (1 µmole) d'ATIII humaine avec 1,7 mg (1 µmole) de pentasaccharide dans 10 ml de tampon NaCl 0.15 M, Tris-HCl 0,01 M, pH 7,5 et on laisse reposer environ 15 mn à température ambiante.

Le pentasaccharide mis en oeuvre est obtenu selon le procédé de synthèse décrit dans l'exemple 9 de la demande de brevet FR 82 18003 au nom de la Demanderesse.

On vérifie la formation du complexe ATIII-pentasaccharide en soumettant un aliquot du mélange obtenu à une opération de gel-filtration et en mesurant la densité optique à 280 nm de fractions éluées.

On utilise une colonne de Séphadex G50 fine (dérivé du dextrane commercialisé par Pharmacie Suède) de 1 × 95 cm, équilibrée avec le tampon ci-dessus. On dépose au sommet de la colonne le mélange d'ATIII et de pentasaccharide. La colonne est ensuite éluée avec ce même tampon et l'on recueille des fractions de 1 ml.

Sur la figure 1, on a représenté la variation de la densité optique DO à 280 nm et le dosage d'acide uroniques à la DO 530 nm en fonction du volume élué pour l'ATIII seul, le pentasaccharide et le complexe ATIII-pentasaccharide recueilli.

L'examen de cette figure montre que les volumes d'élution de l'ATIII et du pentasaccharide sont respectivement de 27 et 48 ml.

Avec le complexe ATIII-pentasaccharide, on n'observe qu'un pic à 27 ml et pas de pic à 48 ml. Lorsqu'on répète l'expérience avec un excès de pentasaccharide, par exemple avec 6,5 mg d'ATIII et 0,313 mg de pentasaccharide, l'excès d'oligosaccharide est élué à 48 ml ce qui prouve la formation d'un complexe équimoléculaire et, par conséquent, la présence d'un seul site de fixation sur le pentasaccharide.

On détermine la constante d'association du complexe par fluorimétrie.

On mesure l'effet du pentasaccharide sur l'augmentation de fluorescence de résidus de tryptophane dans l'ATIII à l'aide d'un spectrofluorimètre de type FICA 55 MK II.

Ces mesures sont effectuées à 25°C dans un tampon tris -HCl 0,01 M pH 7,5 NaCl 0,15 M.

Les longueurs d'ondes d'excitation et d'émission sont respectivement de 278 et 335 nm.

On constate que l'addition de pentasaccharide à l'ATIII augmente la fluorescence de la protéine vraisemblablement au niveau des résidus de tryptophane.

Sur la figure 2 on a rapporté une courbe de la variation du rapport

$$\frac{\bar{v}}{c}$$

du nombre $\bar{v}$ de moles de pentasaccharide lié par mole d'ATIII à la molarité $c$ de pentasaccharide en fonction de $\bar{v}$ (courbe de Scatchard) pour le complexe considéré.

La pente de la droite obtenue permet de calculer la constante d'association de $7 \times 10^6\ M^{-1}$. L'intersection de la courbe avec l'axe des $x$ s'effectue pratiquement à $\bar{v} = 1$, ce qui confirme la présence d'un seul site de fixation.

L'activité anti-Xa est mesurée selon la méthode de Yin et Al par le test amidolytique de Teien en utilisant du tampon tris-maléate 0,02 M pH 7,5 pour la dilution du plasma au lieu de tampons Tris-HCl.

On observe une activité anti-Xa respectivement de l'ordre de 9 000 u/mg de pentasaccharide (YW).

L'activité anti-thrombique mesurée selon le test amidolytique de Larsen est nulle.

Le complexe équimolaire pentasaccharide/AT III possède une action antithrombotique supérieure à celle du pentasaccharide seul, déterminée par la mesure de la génération du fibrinopeptide *A* (méthode selon Emanuele, R. M. et Coll. Fred. Proc. 43 (4) 751). In vivo chez le singe, le complexe équimolaire pentasaccharide/AT III a une demie-vie plus longue, lorsqu'il est administré par voie sous-cutanée ou intraveineuse, à 25 et 50 micro/kg que celles du pentasaccharide seul et de l'AT III seule.

De même, par voie intraveineuse, il a une activité antithrombotique supérieure à celle du pentasaccharide seul, mesurée sur le modèle de thrombose par stase, selon la méthode de ANDERSEN, A., et Coll. (1981) Thrombos. and Hemost. 46(1), 117, en utilisant comme agent thrombogène PCC/RVV et FEIBA.

Exemple 2

Préparation d'un complexe ATIII-hexasaccharide de type CDEFGH où DEFGH correspondant à la séquence de l'exemple 1:

On opère, comme décrit dans l'exemple 1 et en préparant l'hexasaccharide selon le procédé de synthèse décrit dans la demande de brevet FR ci-dessus. On obtient un complexe à forte constante d'association dont l'activité spécifique anti-Xa est également largement supérieure à celle de l'hexasaccharide seul.

## Revendications

1. Complexes contenant des oligosaccharides à activité anti-Xa élevée, caractérisés en ce qu'il s'agit de complexes formés, selon des quantités équimolaires, d'AT III et d'oligosaccharides de formule:

$$X\ (aU)_n\ Y$$

dans laquelle:

*X* est un groupe —OH ou une structure acide uronique soit *b* de type acide D-glucuronique, soit *c* de type acide L-iduronique,

*Y* est un groupe —OH ou une structure acide aminé de type D-glucosamine,

*a* est une structure acide aminé de type D-glucosamine,

*U* est une structure acide uronique soit *b* de type acide D-glucuronique, soit *c* de type acide L-iduronique, les motifs *a* et *U* formant des chaînes régulières,

*n* est un entier de 2 à 6,

les significations de X, Y et *n* étant telles que le nombre total de motifs saccharidiques est de 5 à 12.

2. Complexes selon revendication 1, caractérisés en ce que les oligosaccharides sont des produits purs tels qu'obtenus par voie de synthèse.

3. Complexes selon l'une quelconque des revendications précédentes, caractérisés en ce que l'alternance régulière des motifs *a* et *U* est modifiée par la présence, à la suite, d'un ou plusieurs motifs *a* ou *U* et/ou d'un ou plusieurs motifs de sucres neutres et/ou de désoxy-sucres et/ou de sucres de configuration différente.

4. Complexes selon l'une quelconque des revendications 1 à 3, caractérisés en ce qu'ils comportent des oligosaccharides comportant ou correspondant, en totalité ou en partie, à une séquence octasaccharidique de type b-a-c-a-b-a-c-a.

5. Complexes selon la revendication 4, caractérisés en ce que dans les oligosaccharides, les groupes alcool primaire et alcool secondaire sont libres, ou sont en partie ou en totalité substitués par des anions tels que des anions phosphate ou en particulier sulfate, notamment sous forme de sel avec un cation minérale ou organique, en particulier un cation alcalin tel que le sodium et/ou que les groupes amino des motifs *a* sont libres ou substitués par des groupes identiques ou différents comprenant les groupes acyle, en particulier acétyle, ou des anions tels que définis ci-dessus, ces différents anions étant avantageusement sous forme de sels avec un cation tel que décrit plus haut et/ou que les groupes carboxyle des motifs *b* ou *c* sont libres ou se présentent de préférence sous forme de sels avec un cation organique ou minéral comme défini plus haut.

6. Complexes contenant des oligosaccharides à activité anti-Xa élevée, caractérisés en ce qu'il s'agit de complexes formés, selon des quantités équimolaires, d'AT III et d'oligosaccharides comprenant en totalité ou en partie la séquence octasaccharidique de structure ABCDEFGH:

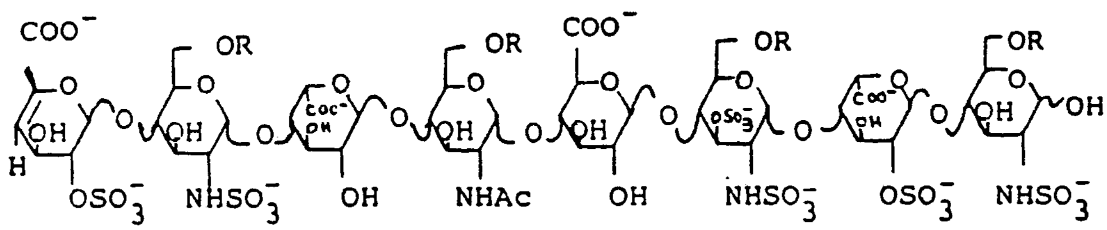

A B C D E F G H

R représentant un atome d'hydrogène ou $—SO_3^-$.

7. Complexes selon la revendication 6, caractérisés en ce qu'il s'agit de complexe AT III-hexasaccharide en particulier de structure de type CDEFGH.

8. Complexes selon la revendication 5, caractérisés en ce qu'ils comportent des pentasaccharides de structure de type a-b-a-c-a sous forme de complexe avec l'AT III.

9. Complexes selon l'une quelconque des revendications précédentes, caractérisés en qu'il s'agit d'un complexe d'AT III et d'un oligosaccharide choisi parmi ceux définis dans les revendications précédentes, totalement dépourvu d'activité anti-IIa.

10. Complexes caractérisés en ce qu'il s'agit d'un complexe d'AT III humaine et de pentasaccharide de formule:

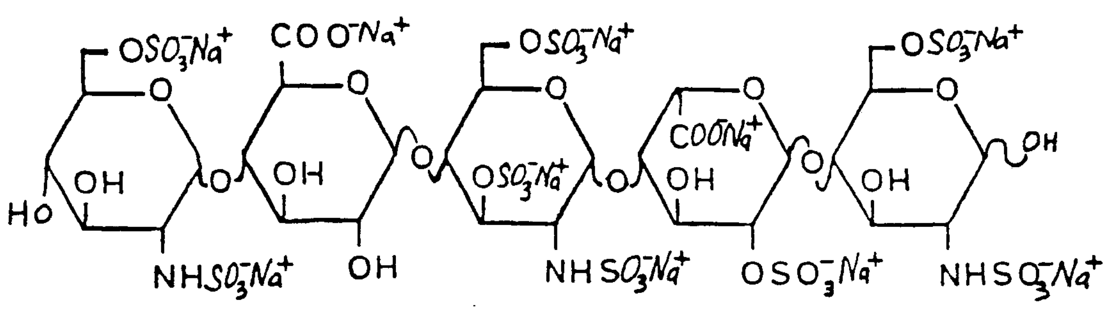

11. Composition pharmaceutique, caractérisée en ce qu'elle renferme une quantité efficade d'un complexe selon l'une quelconque des revendications 1 à 10 en association avec un excipient pharmaceutique.

12. Composition pharmaceutique selon la revendication 11, caractérisée en ce qu'elle se présente sous forme lyophilisée, ou sous une forme appropriée soit pour l'administration par voie orale, notamment, sous forme de gélules gastro-résistantes, de comprimés ou tablettes, de pilules ou de liposomes, soit pour

l'administration par voie rectale plus spécialement sous forme de suppositoires, soit encore sous forme de solutions administrables par voie intraveineuse, intramusculaire ou sous-cutanée ou sous forme d'aérosols ou de pommade.

13. Composition pharmaceutique selon la revendication 12, caractérisée en ce que les solutions destinées à l'administration par voie intraveineuse, renferment 1000 à 50000 u (YW)/m, de préférence de 1500 à 30000, notamment 25000 u/ml d'oligosaccharides.

**Patentansprüche**

1. Komplexe, die Oligosaccharide mit erhöhter Anti-Xa-Aktivität enthalten, dadurch gekennzeichnet, daß es sich um Komplexe aus äquimolaren Mengen an AT III und Oligosacchariden der Formel:

$$X\,(aU)_n\,Y$$

handelt, worin:

*X* eine OH-Gruppe oder eine Uronsäurestruktur entweder vom Typ D-Glukuronsäure oder vom Typ L-Iduronsäure darstellt,

*Y* eine OH-Gruppe oder eine Aminosäurestruktur vom Typ D-Glukosamin darstellt,

*a* eine Aminosäurestruktur vom Typ D-Glukosamin ist,

*U* eine Uronsäurestruktur entweder vom Typ D-Glukuronsäure oder vom Typ L-Iduronsäure darstellt, wobei die Gruppen *a* und *U* regelmäßige Ketten bilden,

*n* eine ganze Zahl von 2 bis 6 bedeutet, und

X, Y und *n* die Bedeutung haben, daß die Gesamtzahl an Saccharidgruppen 5 bis 12 beträgt.

2. Komplexe nach Anspruch 1, dadurch gekennzeichnet, daß die Oligosaccharide Reinprodukte sind, wie sie durch Synthese erhalten werden.

3. Komplexe nach einem der vorstehenden Ansprüche, dadurch gekennzeichnet, daß die regelmäßige Abfolge der Gruppen *a* und *U* der Gegenwart einer oder mehrerer Gruppen *a* oder *U* und/oder einer oder mehrerer Gruppen von Neutralzuckern und/oder Desoxyzuckern und/oder Zuckern verschiedener Konfiguration abgeändert wird.

4. Komplexe nach einem der Ansprüche 1 bis 3, dadurch gekennzeichnet, daß sie Oligosaccharide umfassen, welche ihrerseits in ihrer Ganzheit oder teilweise eine Abfolge von Octasacchariden des Typs b-a-c-a-b-a-c-a umfassen oder dieser entsprechen.

5. Komplexe nach Anspruch 4, dadurch gekennzeichnet, daß die primären und sekundären Alkoholgruppen in den Oligosacchariden frei sind oder teilweise oder vollständig durch Anionen wie Phosphatanionen oder insbesondere Sulfatanionen ersetzt sind, ganz besonders in Form eines Salzes mit einem Mineral- oder organischem Kation, insbesondere einem Alkalikation wie dem Natriumion, und/oder daß die Aminogruppen der Gruppen *a* frei sind oder durch identische oder verschiedene Gruppen, umfassend die Acylgruppierungen, insbesondere die Acetylgruppe, oder durch Anionen wie vorstehend definiert, ersetzt sind, wobei die verschiedenen Anionen vorteilhafterweise in Form ihrer Salze mit einem Kation wie oben beschrieben vorliegen, und/oder daß die Carboxylgruppen der Gruppen b oder c frei sind oder vorzugsweise in Form ihrer Salze mit einem organischen oder mit einem Mineralkation wie vorstehend definiert, vorliegen.

6. Komplexe, die Oligosaccharide mit erhöhter Anti-Xa-Aktivität enthalten, dadurch gekennzeichnet, daß es sich um Komplexe aus äquimolaren Mengen von AT III und Oligosacchariden handelt, welche in ihrer Ganzheit oder teilweise die Abfolge von Octasacchariden der Struktur ABCDEFGH umfassen:

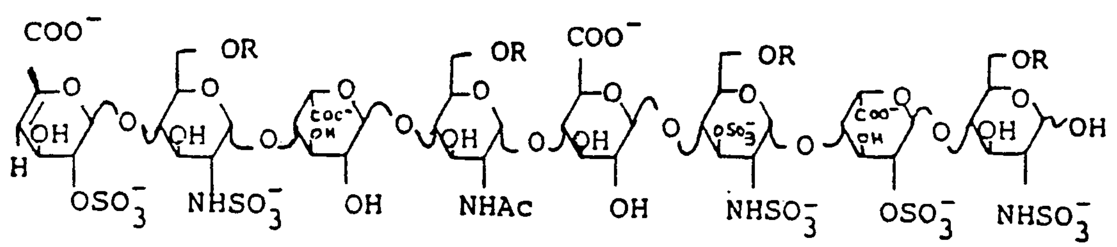

A B C D E F G H

wobei R ein Wasserstoffatom oder den Rest $-SO_3^-$ darstellt.

7. Komplexe nach Anspruch 6, dadurch gekennzeichnet, daß es sich um einen Komplex aus AT III und einem Hexasaccharid, insbesondere einem mit der Struktur des Typs CDEFGH, handelt.

8. Komplexe nach Anspruch 5, dadurch gekennzeichnet, daß sie Pentasaccharide der Struktur vom Typ a-b-a-c-a in Form ihrer Komplexe mit AT III umfassen.

9. Komplex nach einem der vorstehenden Ansprüche, dadurch gekennzeichnet, daß es sich um einen Komplex von AT III mit einem wie in den vorstehenden Ansprüchen definierten ausgewählten Oligosacharid handelt, welches vollständig ohne Anti-Xa-Aktivität ist.

10. Komplexe, dadurch gekennzeichnet, daß es sich um Komplexe aus menschlichem AT III und Pentasacharid der Formel:

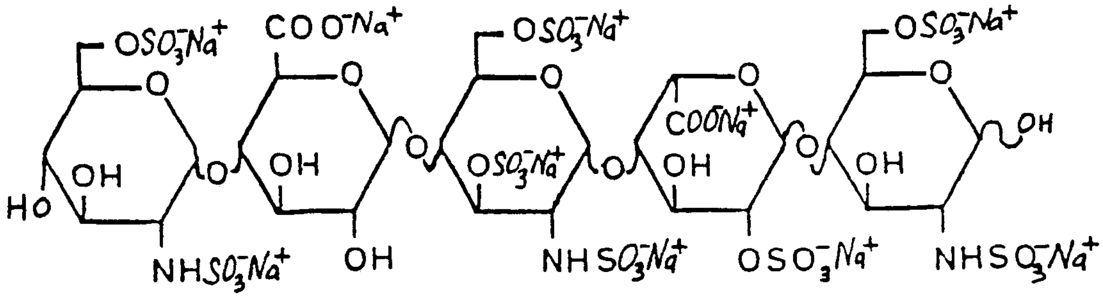

handelt.

11. Pharmazeutische Zusammensetzung, dadurch gekennzeichnet, daß sie eine wirksame Menge eines Komplexes gemäß einen der Ansprüche 1 bis 10 in Verbindung mit einem pharmazeutischen Exzipienz enthält.

12. Pharmazeutische Zusammensetzung nach Anspruch 11, dadurch gekennzeichnet, daß sie in lyophilisierter Form oder in einer für die orale Verabreichung geeigneten Form, insbesondere in Form von magenresistenten Gelatinekapseln, in Form von Komprimaten oder Tabletten, Pillen oder Liposomen, oder für die rektale Verabreichung vorwiegend in Form von Suppositorien, oder auch in Form von Lösungen für die intravenöse, intramuskuläre oder subkutane Verabreichung oder in Form von Aerosolen oder Salben vorliegt.

13. Pharmazeutische Zusammensetzung nach Anspruch 12, dadurch gekennzeichnet, daß die zur intravenösen Verabreichung vorgesehenen Lösungen 1000 bis 50 000 u (YW)/ml vorzugsweise von 1500 bis 30 000, insbesondere 25 000 u (YW)/ml enthalten.

**Claims**

1. Complexes containing oligosaccharides with high anti-Xa activity, characterised in that they are complexes composed, in equimolar amounts, of ATIII and oligosaccharides of formula:

$$X\ (aU)_n\ Y$$

in which:

*X* is a —OH group or a uronic acid structure of either the D-glucuronic acid type or the L-iduronic acid type,

*Y* is a —OH group or an aminated acid structure of the D-glucosamine type,

*a* is an aminated acid structure of the D-glucosamine type,

*U* is a uronic acid structure of either the D-glucuronic acid type or the L-iduronic acid type, the residues *a* and *U* forming regular chains,

*n* is an integer from 2 to 6,

the meanings of X, Y and *n* being such that the total number of sugar residues is 5 to 12.

2. Complexes according to Claim 1, characterised in that the oligosaccharides are pure products such as obtained by the synthetic route.

3. Complexes according to any one of the preceding Claims, characterised in that the regular alternation of the residues *a* and *U* is modified by the presence, one after the other, of one or several *a* or *U* residues and/or one or several residues of neutral sugars and/or deoxy sugars and/or sugars of different configuration.

4. Complexes according to any one of the Claims 1 to 3, characterised in that they comprise oligosaccharides containing or corresponding to, in whole or in part, an octasaccharide sequence of the b-a-c-a-b-a-c-a type.

5. Complexes according to Claim 4, characterised in that in the oligosaccharides, the primary alcohol groups and the secondary alcohol groups are free, or are in part or in their entirety substituted by anions such as phosphate or, in particular, sulfate anions, in particular in the form of a salt with a mineral or organic cation, in particular an alkali cation such as sodium and/or that the amino groups of the *a* residues are free or substituted by identical or different groups including acyl groups, in particular acetyl, or anions such as defined above, these different anions being advantageously in the form of salts with a cation as described above and/or that the carboxyl groups of the *b* or *c* residues are free or are present preferably in the form of salts with an organic or mineral cation as defined above.

6. Complexes containing oligosaccharides with high anti-Xa activity, characterised in that they are complexes composed, in equimolar amounts, of ATIII and oligosaccharides comprising as a whole or in part the octasaccharide sequence of structure ABCDEFGH:

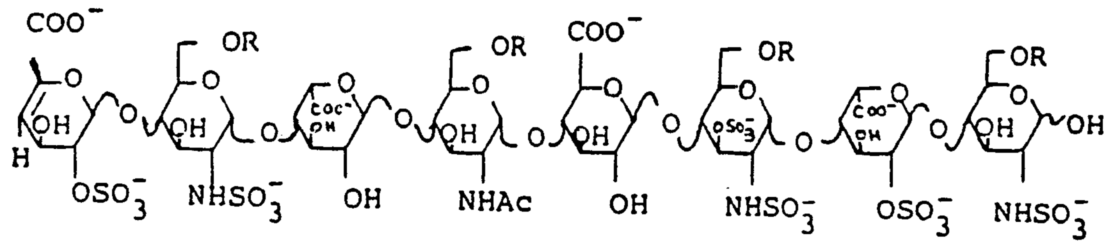

A B C D E F G H

R representing a hydrogen atom or $—SO_3^-$.

7. Complexes according to Claim 6, characterised in that they are ATIII-hexasaccharide complexes, in particular of structure of the CDEFGH type.

8. Complexes according to Claim 5, characterised in that they include pentasaccharides of structure of the a-b-a-c-a type in the form of a complex with ATIII.

9. Complexes according to any one of the preceding Claims, characterised in that it concerns a complex of ATIII and an oligosaccharide selected from those defined in the preceding Claims, totally devoid of anti-IIa activity.

10. Complexes characterised in that it concerns a complex of human ATIII and the pentasaccharide of formula:

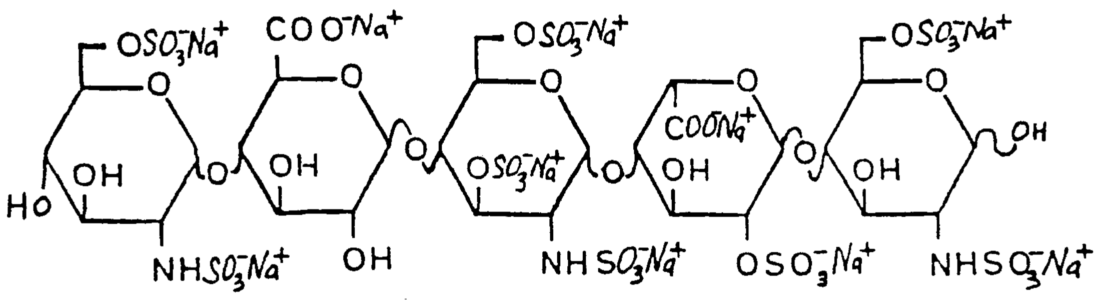

11. Pharmaceutical composition, characterised in that it contains an efficacious quantity of a complex according to any one of the Claims 1 to 10 in combination with a pharmaceutical excipient.

12. Pharmaceutical composition according to Claim 11, characterised in that it is available in lyophilized form, or in a form suitable either for administration by the oral route, in particular, in the form of gastro-resistant capsules, tablets or lozenges, pills or liposomes, or for administration by the rectal route, more particularly in the form of suppositories, or also in the form of solutions administrable by the intravenous, intramuscular or subcutaneous route or in the form of aerosols or ointments.

13. Pharmaceutical compositions according to Claim 12, characterised in that the solutions intended for administration by the intravenous route contain 1000 to 50000 u (YW)/ml, preferably 1500 to 30000, and in particular 25000 u/ml of oligosaccharides.

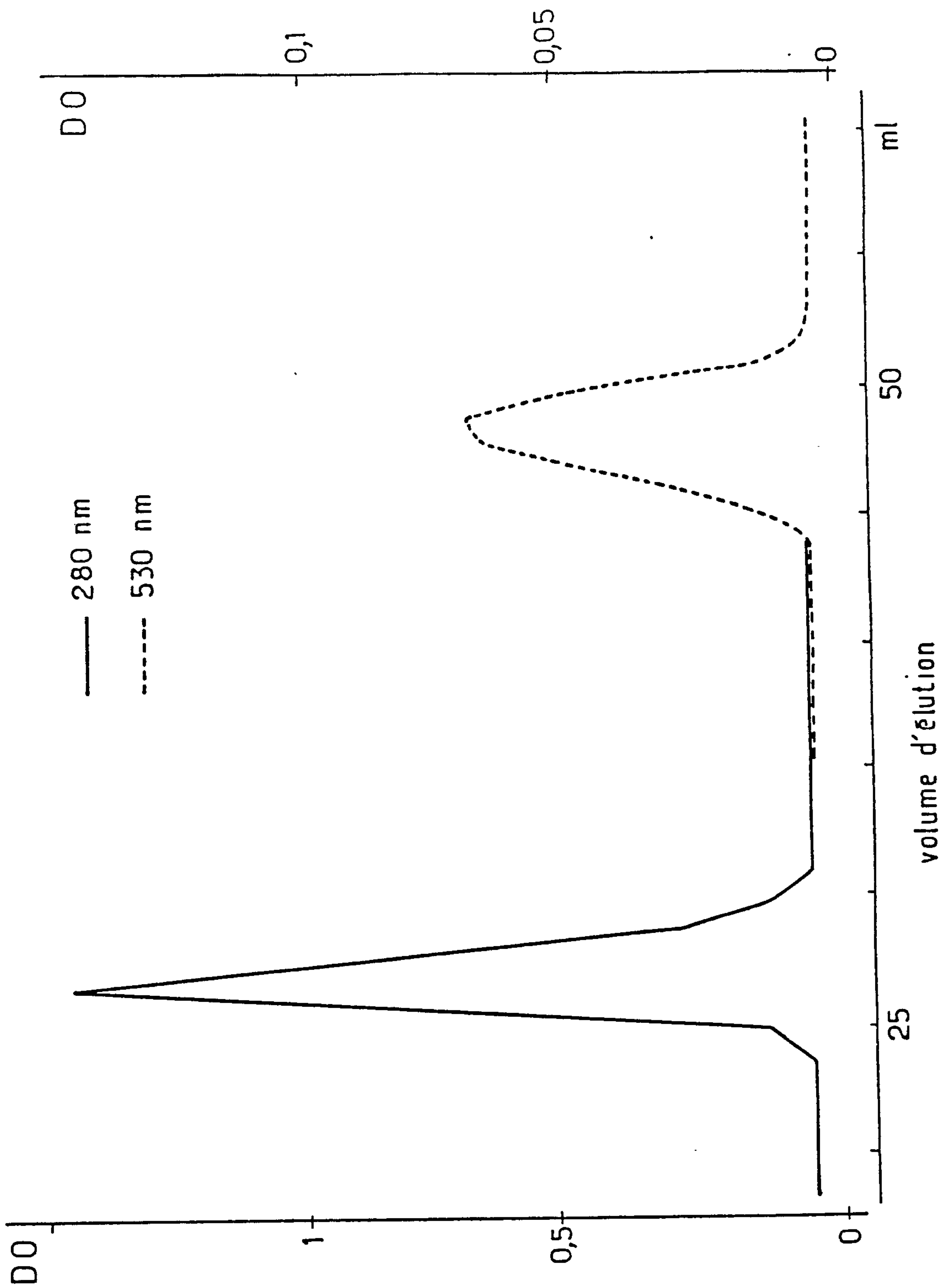


FIG.1.

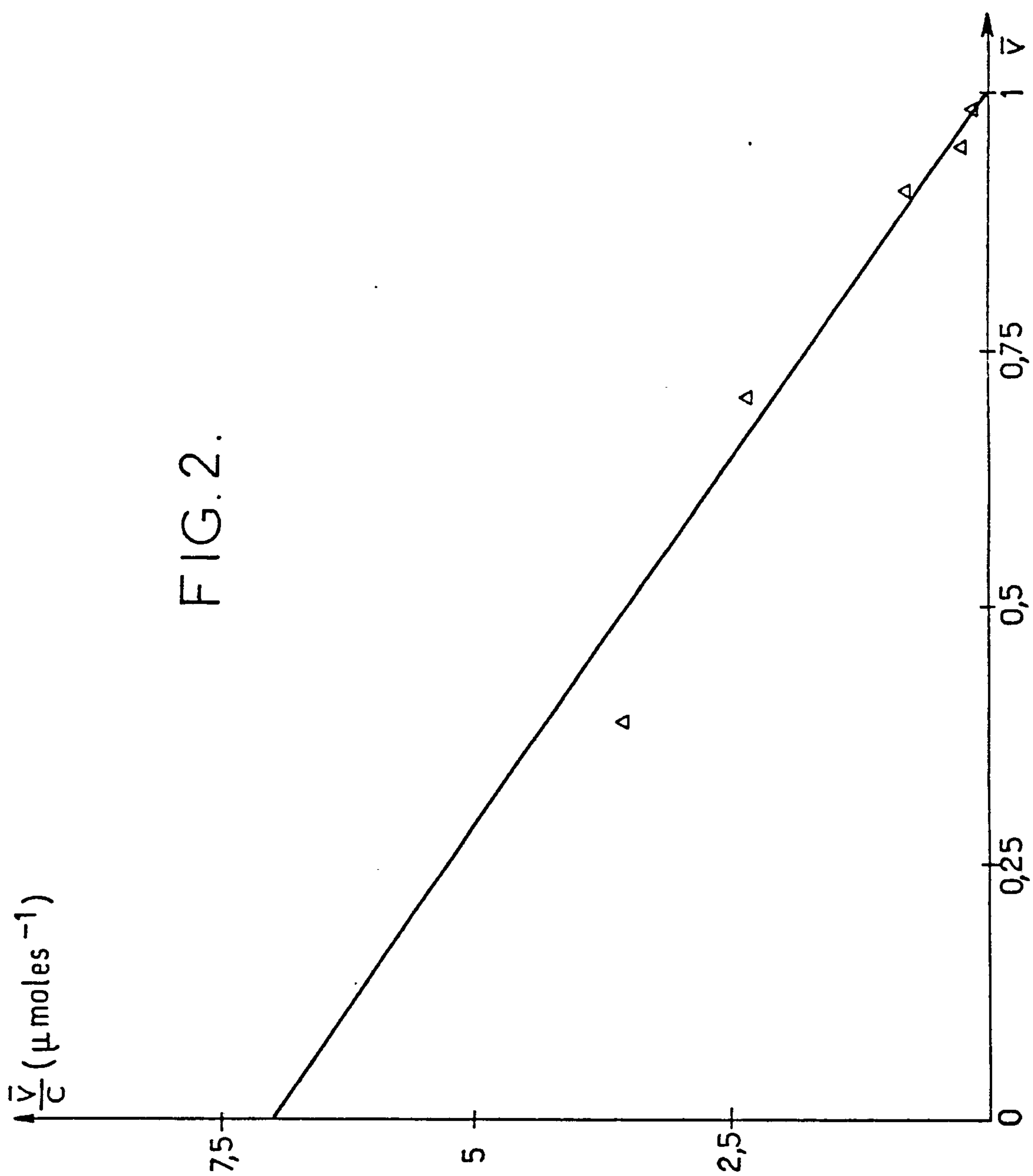


FIG. 2.